# EUROPÄISCHE PATENTSCHRIFT

(11) **EP 1 812 365 B1**
(45) Veröffentlichungstag und Bekanntmachung des Hinweises auf die Patenterteilung: **21.05.2008**
(21) Anmeldenummer: 05806496.5
(22) Anmeldetag: 11.11.2005
(51) Int. Cl.: C07C 5/333, C07C 11/167

(54) **VERFAHREN ZUR HERSTELLUNG VON BUTADIEN AUS N-BUTAN**
METHOD FOR PRODUCING BUTADIENE FROM N-BUTANE
PROCEDE POUR PRODUIRE DU BUTADIENE A PARTIR DE N-BUTANE

(30) Priorität: 12.11.2004 DE 102004054766
(43) Veröffentlichungstag der Anmeldung: 01.08.2007
(73) Patentinhaber: BASF SE, 67056 Ludwigshafen (DE)
(72) Erfinder: CRONE, Sven, 67117 Limburgerhof (DE); KLANNER, Catharina, 68163 Mannheim (DE); SCHINDLER, Götz-Peter, 68219 Mannheim (DE); DUDA, Mark, 67071 Ludwigshafen (DE); BORGMEIER, Frieder, 68163 Mannheim (DE)
(74) Vertreter: Isenbruck, Günter
(86) Internationale Anmeldenummer: PCT/EP2005/012109
(87) Internationale Veröffentlichungsnummer: WO 2006/050969

(56) Entgegenhaltungen:
- WO-A-20/04007408
- US-A- 4 595 788
- US-A- 4 718 986
- US-A1- 2005 171 311

## Beschreibung

Die Erfindung betrifft ein Verfahren zur Herstellung von Butadien aus n-Butan.

Butadien ist eine bedeutende Grundchemikalie und wird beispielsweise zur Herstellung von Synthesekautschuken (Butadien-Homopolymere, Styrol-Butadien-Kautschuk oder Nitril-Kautschuk) oder zur Herstellung von thermoplastischen Terpolymeren (AcrylnitrilButadien-Styrol-Copolymere) eingesetzt. Butadien wird ferner zu Sulfolan, Chloropren und 1,4-Hexamethylendiamin (über 1,4-Dichlorbuten und Adipinsäuredinitril) umgesetzt. Durch Dimerisierung von Butadien kann ferner Vinylcyclohexen erzeugt werden, welches zu Styrol dehydriert werden kann.

Butadien kann durch thermische Spaltung (Steam-Cracken) gesättigter Kohlenwasserstoffe hergestellt werden, wobei üblicherweise von Naphtha als Rohstoff ausgegangen wird. Beim Steam-Cracken von Naphtha fällt ein Kohlenwasserstoff-Gemisch aus Methan, Ethan, Ethen, Acetylen, Propan, Propen, Propin, Allen, Butenen, Butadien, Butinen, Methylallen, C₅- und höheren Kohlenwasserstoffen an.

Nachteilig an der Erzeugung von Butadien im Crackprozess ist, dass zwangsläufig größere Mengen an unerwünschten Koppelprodukten anfallen.

Aufgabe der Erfindung ist es, ein Verfahren zur Herstellung von Butadien aus n-Butan bereitzustellen, bei dem in möglichst geringem Umfang Koppelprodukte anfallen.

Gelöst wird die Aufgabe durch ein Verfahren zur Herstellung von Butadien aus n-Butan mit den Schritten:
A) Bereitstellung eines n-Butan enthaltenden Einsatzgasstroms a;
B) Einspeisung des n-Butan enthaltenden Einsatzgasstroms a in mindestens eine erste Dehydrierzone und nicht-oxidative, katalytische Dehydrierung von n-Butan, wobei ein Gasstrom b enthaltend n-Butan, 1-Buten, 2-Buten, Butadien, Wasserstoff, gegebenenfalls Kohlendioxid und gegebenenfalls Wasserdampf erhalten wird;
C) Einspeisung des Gasstroms b und eines sauerstoffhaltigen Gases in mindestens eine zweite Dehydrierzone und oxidative Dehydrierung von 1-Buten und 2-Buten, wobei ein Gasstrom enthaltend n-Butan, Butadien, Wasserstoff, Kohlendioxid und Wasserdampf erhalten wird,
D) Kompression in mindestens einer ersten Kompressionsstufe und Abkühlung des Gasstroms c, wobei mindestens ein Kondensatstrom d1 enthaltend Wasser und ein Gasstrom d2 enthaltend n-Butan, Butadien, Wasserstoff, Kohlendioxid und Wasserdampf erhalten wird,
E) Kompression in mindestens einer weiteren Kompressionsstufe und Abkühlung des Gasstroms d2, wobei mindestens ein Kondensatstrom e1 enthaltend n-Butan, Butadien und Wasser und ein Gasstrom e2 enthaltend n-Butan, Butadien, Wasserstoff und Kohlendioxid erhalten werden,
F) Abkühlung des Produktgasstroms e2, wobei ein Kondensatstrom f1 enthaltend n-Butan und Butadien und ein Abgasstrom f2 enthaltend Kohlendioxid und Wasserstoff erhalten werden,
G) Abtrennung von Wasser aus dem mindestens einen Kondensatstrom e1 und gegebenenfalls dem Kondensatstrom f1 durch Phasentrennung, wobei ein C₄-Kohlenwasserstoffstrom g1 enthaltend n-Butan und Butadien und mindestens ein Abwasserstrom g2 erhalten werden,
H) Auftrennung des C₄-Kohlenwasserstoffstroms g1 in einen n-Butan enthaltenden Rückführstrom h1 und einen im Wesentlichen aus Butadien bestehenden Produktstrom h2 und Rückführung des Stroms h1 in die erste Dehydrierzone.

Das erfindungsgemäße Verfahren zeichnet sich durch eine besonders effektive Ausnutzung der Rohstoffe aus. So werden Verluste des Rohstoffs n-Butan durch Rückführung von nicht umgesetztem n-Butan in die Dehydrierung minimiert. Durch die Koppelung von nicht-oxidativer katalytischer Dehydrierung und oxidativer Dehydrierung wird eine hohe Butadien-Ausbeute erzielt. Das Verfahren ist im Vergleich zur Erzeugung von Butadien durch Cracken durch eine hohe Selektivität gekennzeichnet. Es fallen keine Koppelprodukte an. Es entfällt die aufwendige Abtrennung von Butadien aus dem Produktgasgemisch des Crackprozesses.

In einem ersten Verfahrensteil A wird ein n-Butan enthaltender Einsatzgasstrom a bereitgestellt. Üblicherweise wird dabei von an n-Butan reichen Gasgemischen wie liquefied petroleum gas (LPG) als Rohstoff ausgegangen. LPG enthält im Wesentlichen gesättigte C₂-C₅-Kohlenwasserstoffe. Daneben enthält es auch Methan und Spuren von C₆⁺-Kohlenwasserstoffen. Die Zusammensetzung von LPG kann stark schwanken. Vorteilhafter Weise enthält das eingesetzte LPG mindestens 10 Gew.-% Butane.

Alternativ kann ein veredelter C₄-Strom aus Crackern oder Raffinerien eingesetzt werden.

In einer Variante des erfindungsgemäßen Verfahrens umfasst die Bereitstellung des n-Butan enthaltenden Dehydrier-Einsatzgasstromes die Schritte
(A1) Bereitstellung eines liquefied petroleum gas (LPG)-Stroms,
(A2) Abtrennung von Propan und gegebenenfalls Methan, Ethan und C₅⁺-Kohlenwasserstoffen (hauptsächlich Pentane, daneben Hexane, Heptane, Benzol, Toluol) aus dem LPG-Strom, wobei ein Butane (n-Butan und Isobutan) enthaltender Strom erhalten wird,
(A3) Abtrennung von Isobutan aus dem Butane enthaltenden Strom, wobei der n-Butan enthaltende Einsatzgasstrom erhalten wird, und gegebenenfalls Isomerisierung des abgetrennten Isobutans zu einem n-Butan/Isobutan-Gemisch und Rückführung des n-Butan/Isobutan-Gemischs in die Isobutan-Abtrennung.

Die Abtrennung von Propan und gegebenenfalls Methan, Ethan und C₅⁺-Kohlenwasserstoffen erfolgt beispielsweise in einer oder mehreren üblichen Rektifizierkolonnen. Beispielsweise können in einer ersten Kolonne Leichtsieder (Methan, Ethan, Propan) über Kopf und in einer zweiten Kolonne Schwersieder (C₅⁺-Kohlenwasserstoffe) am Kolonnensumpf abgetrennt werden. Es wird ein Butane (n-Butan und Isobutan) enthaltender Strom erhalten, aus dem Isobutan beispielsweise in einer üblichen Rektifizierkolonne abgetrennt wird. Der verbleibende, n-Butan enthaltende Strom wird als Einsatzgasstrom für die nachfolgende Butan-Dehydrierung eingesetzt.

Der abgetrennte Isobutan-Strom kann einer Isomerisierung unterworfen. Dazu wird der Isobutan enthaltende Strom in einen Isomerisierungsreaktor eingespeist. Die Isomerisierung von Isobutan zu n-Butan kann wie in GB-A 2 018 815 beschrieben durchgeführt werden. Es wird ein n-Butan/Isobutan-Gemisch erhalten, das in die n-Butan/Isobutan-Trennkolonne eingespeist wird.

Der abgetrennte Isobutan-Strom kann auch einer weiteren Verwendung zugeführt werden, beispielsweise zur Herstellung von Methacrylsäure, Polyisobuten oder Methyl-tert.-butylether eingesetzt werden.

Der n-Butan enthaltende Einsatzgasstrom a enthält im Allgemeinen mindestens 60 Gew.-% n-Butan, vorzugsweise mindestens 90 Gew.-% n-Butan. Daneben kann er als Nebenbestandteile noch C₁-C₆-Kohlenwasserstoffe enthalten.

In einem Verfahrensteil B wird der n-Butan enthaltende Einsatzgasstrom in eine Dehydrierzone eingespeist und einer nicht-oxidativen katalytischen Dehydrierung unterworfen. Dabei wird n-Butan in einem Dehydrierreaktor an einem dehydrieraktiven Katalysator teilweise zu 1-Buten und 2-Buten dehydriert, wobei auch Butadien gebildet wird. Daneben fallen Wasserstoff und in geringen Mengen Methan, Ethan, Ethen, Propan und Propen an. Je nach Fahrweise der Dehydrierung können außerdem Kohlenstoffoxide (CO, CO₂), Wasser und Stickstoff im Produktgasgemisch der nicht-oxidativen katalytischen n-Butan-Dehydrierung enthalten sein. Daneben liegt im Produktgasgemisch nicht umgesetztes n-Butan vor.

Die nicht-oxidative katalytische n-Butan-Dehydrierung kann mit oder ohne sauerstoffhaltigem Gas als Co-Feed durchgeführt werden. Vorzugsweise wird sie als autotherme nicht-oxidative katalytische Dehydrierung unter Einspeisung von Sauerstoff als Co-Feed durchgeführt. Bei der autothermen Fahrweise wird die benötigte Wärme direkt im Reaktorsystem durch Verbrennung von Wasserstoff und/oder Kohlenwasserstoffen in Gegenwart von Sauerstoff erzeugt. Gegebenenfalls kann zusätzlich ein Wasserstoff enthaltender Co-Feed zugemischt werden. Sauerstoff kann als Rein-Sauerstoff oder als sauerstoffhaltiges Gas eingespeist werden, wobei der Sauerstoffgehalt aber mindestens 75 Vol.%, vorzugsweise mindestens 90 Vol.-% beträgt. Ein geeignetes sauerstoffhaltiges Gas ist technisch reiner Sauerstoff mit einem Sauerstoffgehalt von ca. 99 Vol.%. Durch die Verwendung von einem sauerstoffhaltigen Co-Feed mit hohem Sauerstoffgehalt werden nur geringe Mengen an Inertgasen (Stickstoff) in den Gesamtprozess eingeschleust. Dadurch kann die Trennung der C₄-Kohlenwasserstoffe von Wasserstoff und Kohlendioxid leicht durch Auskondensieren der C₄-Kohlenwasserstoffe erfolgen. Wegen des geringen Inertgasanteils in dem Gasstrom e2 bleibt der Verlust an C₄-Kohlenwasserstoffen in dem Kondensationsschritt F) möglichst niedrig.

Ein Merkmal der nicht-oxidativen Fahrweise gegenüber einer oxidativen Fahrweise ist, dass bei der oxidativen Dehydrierung kein freier Wasserstoff in wesentlichen Mengen gebildet wird.

Die nicht-oxidative katalytische n-Butan-Dehydrierung kann grundsätzlich in allen aus dem Stand der Technik bekannten Reaktortypen und Fahrweisen durchgeführt werden. Eine vergleichsweise ausführliche Beschreibung von erfindungsgemäß geeigneten Dehydrierverfahren enthält auch "Catalytica^{®} Studies Division, Oxidative Dehydrogenation and Alternative Dehydrogenation Processes" (Study Number 4192 OD, 1993, 430 Ferguson Drive, Mountain View, California, 94043-5272, USA).

Eine geeignete Reaktorform ist der Festbettrohr- oder Rohrbündelreaktor. Bei diesen befindet sich der Katalysator (Dehydrierungskatalysator und, bei Arbeiten mit Sauerstoff als Co-Feed, gegebenenfalls spezieller Oxidationskatalysator) als Festbett in einem Reaktionsrohr oder in einem Bündel von Reaktionsrohren. Die Reaktionsrohre werden üblicherweise dadurch indirekt beheizt, dass in dem die Reaktionsrohre umgebenden Raum ein Gas, z.B. ein Kohlenwasserstoff wie Methan, verbrannt wird. Günstig ist es dabei, diese indirekte Form der Aufheizung lediglich auf den ersten ca. 20 bis 30% der Länge der Festbettschüttung anzuwenden und die verbleibende Schüttungslänge durch die im Rahmen der indirekten Aufheizung freigesetzte Strahlungswärme auf die erforderliche Reaktionstemperatur aufzuheizen. Übliche Reaktionsrohr-Innendurchmesser betragen etwa 10 bis 15 cm. Ein typischer Dehydrierrohrbündelreaktor umfasst ca. 300 bis 1000 Reaktionsrohre. Die Temperatur im Reaktionsrohrinneren bewegt sich üblicherweise im Bereich von 300 bis 1200°C, vorzugsweise im Bereich von 500 bis 1000°C. Der Arbeitsdruck liegt üblicherweise zwischen 0,5 und 8 bar, häufig zwischen 1 und 2 bar bei Verwendung einer geringen Wasserdampfverdünnung (analog dem Linde-Verfahren zur Propan-Dehydrierung), aber auch zwischen 3 und 8 bar bei Verwendung einer hohen Wasserdampfverdünnung (analog dem sogenannten "stream active reforming process" (STAR-Prozess) zur Dehydrierung von Propan oder Butan von Phillips Petroleum Co., siehe US 4,902,849, US 4,996,387 und US 5,389,342). Typische Katalysatorbelastungen (GHSV) liegen bei 500 bis 2000 h⁻¹, bezogen auf eingesetzten Kohlenwasserstoff. Die Katalysatorgeometrie kann beispielsweise kugelförmig oder zylindrisch (hohl oder voll) sein.

Die nicht-oxidative katalytische n-Butan-Dehydrierung kann auch, wie in Chem. Eng. Sci. 1992 b, 47 (9-11) 2313 beschrieben, heterogen katalysiert im Wirbelbett durchgeführt werden. Zweckmäßigerweise werden dabei zwei Wirbelbetten nebeneinander betrieben, von denen sich eines in der Regel im Zustand der Regenerierung befindet.

Der Arbeitsdruck beträgt typischerweise 1 bis 2 bar, die Dehydriertemperatur in der Regel 550 bis 600°C. Die für die Dehydrierung erforderliche Wärme wird dabei in das Reaktionssystem eingebracht, indem der Dehydrierkatalysator auf die Reaktionstemperatur vorerhitzt wird. Durch die Zumischung eines Sauerstoff enthaltenden Co-Feeds kann auf die Vorerhitzer verzichtet werden, und die benötigte Wärme direkt im Reaktorsystem durch Verbrennung von Wasserstoff und/oder Kohlenwasserstoffen in Gegenwart von Sauerstoff erzeugt werden. Gegebenenfalls kann zusätzlich ein Wasserstoff enthaltender Co-Feed zugemischt werden.

Die nicht-oxidative katalytische n-Butan-Dehydrierung kann mit oder ohne Sauerstoffhaltigem Gas als Co-Feed in einem Hordenreaktor durchgeführt werden. Vorzugsweise wird sie mit sauerstoffhaltigem Gas als Co-Feed durchgeführt. Dieser enthält ein oder mehrere aufeinanderfolgende Katalysatorbetten. Die Anzahl der Katalysatorbetten kann 1 bis 20, zweckmäßigerweise 1 bis 6, bevorzugt 1 bis 4 und insbesondere 1 bis 3 betragen. Die Katalysatorbetten werden vorzugsweise radial oder axial vom Reaktionsgas durchströmt. Im Allgemeinen wird ein solcher Hordenreaktor mit einem Katalysatorfestbett betrieben. Im einfachsten Fall sind die Katalysatorfestbetten in einem Schachtofenreaktor axial oder in den Ringspalten von konzentrisch angeordneten zylindrischen Gitterrosten angeordnet. Ein Schachtofenreaktor entspricht einer Horde. Die Durchführung der Dehydrierung in einem einzelnen Schachtofenreaktor entspricht einer bevorzugten Ausführungsform, wobei mit sauerstoffhaltigem Co-Feed gearbeitet werden kann. In einer weiteren bevorzugten Ausführungsform wird die Dehydrierung in einem Hordenreaktor mit 3 Katalysatorbetten durchgeführt. Bei einer Fahrweise ohne sauerstoffhaltigem Gas als Co-Feed wird das Reaktionsgasgemisch im Hordenreaktor auf seinem Weg von einem Katalysatorbett zum nächsten Katalysatorbett einer Zwischenerhitzung unterworfen, z.B. durch Überleiten über mit heißen Gasen erhitzte Wärmeaustauscherflächen oder durch Durchleiten durch mit heißen Brenngasen beheizte Rohre.

In einer bevorzugten Ausführungsform des erfindungsgemäßen Verfahrens wird die nicht-oxidative katalytische n-Butan-Dehydrierung autotherm durchgeführt. Dazu wird dem Reaktionsgasgemisch der n-Butan-Dehydrierung in mindestens einer Reaktionszone zusätzlich Sauerstoff zugemischt und der in dem Reaktionsgasgemisch enthaltene Wasserstoff und/oder Kohlenwasserstoff zumindest teilweise verbrannt, wodurch zumindest ein Teil der benötigten Dehydrierwärme in der mindestens einen Reaktionszone direkt in dem Reaktionsgasgemisch erzeugt wird.

Im allgemeinen wird die Menge des dem Reaktionsgasgemisch zugesetzten sauerstoffhaltigen Gases so gewählt, dass durch die Verbrennung von im Reaktionsgasgemisch vorhandenen Wasserstoff und gegebenenfalls von im Reaktionsgasgemisch vorliegenden Kohlenwasserstoffen und/oder von in Form von Koks vorliegendem Kohlenstoff die für die Dehydrierung des n-Butans benötigte Wärmemenge erzeugt wird. Im Allgemeinen beträgt die insgesamt zugeführte Sauerstoffmenge, bezogen auf die Gesamtmenge des Butans, 0,001 bis 0,5 mol/mol, bevorzugt 0,005 bis 0,2 mol/mol, besonders bevorzugt 0,05 bis 0,2 mol/mol. Sauerstoff kann entweder als reiner Sauerstoff oder als sauerstoffhaltiges Gas im Gemisch mit Inertgasen, beispielsweise in Form von Luft, eingesetzt werden. Die Inertgase und die resultierenden Verbrennungsgase wirken im Allgemeinen zusätzlich verdünnend und fördern damit die heterogen katalysierte Dehydrierung.

Der zur Wärmeerzeugung verbrannte Wasserstoff ist der bei der katalytischen n-Butan-Dehydrierung gebildete Wasserstoff sowie gegebenenfalls dem Reaktionsgasgemisch als wasserstoffhaltiges Gas zusätzlich zugesetzter Wasserstoff. Vorzugsweise sollte soviel Wasserstoff zugegen sein, dass das Molverhältnis H₂/O₂ im Reaktionsgasgemisch unmittelbar nach der Einspeisung von Sauerstoff 1 bis 10, bevorzugt 2 bis 5 mol/mol beträgt. Dies gilt bei mehrstufigen Reaktoren für jede Zwischeneinspeisung von sauerstoffhaltigem und gegebenenfalls wasserstoffhaltigem Gas.

Die Wasserstoffverbrennung erfolgt katalytisch. Der eingesetzte Dehydrierungskatalysator katalysiert im Allgemeinen auch die Verbrennung der Kohlenwasserstoffe und von Wasserstoff mit Sauerstoff, so dass grundsätzlich kein von diesem verschiedener spezieller Oxidationskatalysator erforderlich ist. In einer Ausführungsform wird in Gegenwart eines oder mehrerer Oxidationskatalysatoren gearbeitet, die selektiv die Verbrennung von Wasserstoff mit Sauerstoff zu Wasser in Gegenwart von Kohlenwasserstoffen katalysieren. Die Verbrennung dieser Kohlenwasserstoffe mit Sauerstoff zu CO, CO₂ und Wasser läuft dadurch nur in untergeordnetem Maße ab. Vorzugsweise liegen der Dehydrierungskatalysator und der Oxidationskatalysator in verschiedenen Reaktionszonen vor.

Bei mehrstufiger Reaktionsführung kann der Oxidationskatalysator in nur einer, in mehreren oder in allen Reaktionszonen vorliegen.

Bevorzugt ist der Katalysator, der selektiv die Oxidation von Wasserstoff katalysiert, an den Stellen angeordnet, an denen höhere Sauerstoffpartialdrucke herrschen als an anderen Stellen des Reaktors, insbesondere in der Nähe der Einspeisungsstelle für das sauerstoffhaltige Gas. Die Einspeisung von sauerstoffhaltigem Gas und/oder wasserstoffhaltigem Gas kann an einer oder mehreren Stellen des Reaktors erfolgen.

In einer Ausführungsform des erfindungsgemäßen Verfahrens erfolgt eine Zwischeneinspeisung von sauerstoffhaltigem Gas und von wasserstoffhaltigem Gas vor jeder Horde eines Hordenreaktors. In einer weiteren Ausführungsform des erfindungsgemäßen Verfahrens erfolgt die Einspeisung von sauerstoffhaltigem Gas und von wasserstoffhaltigem Gas vor jeder Horde außer der ersten Horde. In einer Ausführungsform ist hinter jeder Einspeisungsstelle eine Schicht aus einem speziellen Oxidationskatalysator vorhanden, gefolgt von einer Schicht aus dem Dehydrierungskatalysator. In einer weiteren Ausführungsform ist kein spezieller Oxidationskatalysator vorhanden. Die Dehydriertemperatur beträgt im allgemeinen 400 bis 1100 °C, der Druck im letzten Katalysatorbett des Hordenreaktors im Allgemeinen 0,2 bis 5 bar, bevorzugt 1 bis 3 bar. Die Belastung (GHSV) beträgt im allgemeinen 500 bis 2000 h⁻¹, bei Hochlastfahrweise auch bis zu 100 000 h⁻¹, bevorzugt 4000 bis 16 000 h⁻¹.

Ein bevorzugter Katalysator, der selektiv die Verbrennung von Wasserstoff katalysiert, enthält Oxide und/oder Phosphate, ausgewählt aus der Gruppe bestehend aus den Oxiden und/oder Phosphaten von Germanium, Zinn, Blei, Arsen, Antimon oder Bismut. Ein weiterer bevorzugter Katalysator, der die Verbrennung von Wasserstoff katalysiert, enthält ein Edelmetall der VIII. und/oder I. Nebengruppe.

Die eingesetzten Dehydrierungskatalysatoren weisen im Allgemeinen einen Träger und eine Aktivmasse auf. Der Träger besteht dabei in der Regel aus einem wärmebeständigen Oxid oder Mischoxid. Bevorzugt enthalten die Dehydrierungskatalysatoren ein Metalloxid, das ausgewählt ist aus der Gruppe bestehend aus Zirkondioxid, Zinkoxid, Aluminiumoxid, Siliciumdioxid, Titandioxid, Magnesiumoxid, Lanthanoxid, Ceroxid und deren Gemischen, als Träger. Bei den Gemischen kann es sich um physikalische Mischungen oder auch um chemische Mischphasen wie Magnesium- oder Zinkaluminiumoxid-Mischoxide handeln. Bevorzugte Träger sind Zirkondioxid und/oder Siliziumdioxid, besonders bevorzugt sind Gemische aus Zirkondioxid und Siliziumdioxid.

Die Aktivmasse der Dehydrierungskatalysatoren enthalten im allgemeinen ein oder mehrere Elemente der VIII. Nebengruppe, bevorzugt Platin und/oder Palladium, besonders bevorzugt Platin. Darüber hinaus können die Dehydrierungskatalysatoren ein oder mehrere Elemente der I. und/oder II. Hauptgruppe aufweisen, bevorzugt Kalium und/oder Cäsium. Weiterhin können die Dehydrierungskatalysatoren ein oder mehrere Elemente der III. Nebengruppe einschließlich der Lanthaniden und Actiniden enthalten, bevorzugt Lanthan und/oder Cer. Schließlich können die Dehydrierungskatalysatoren ein oder mehrere Elemente der III. und/oder IV. Hauptgruppe aufweisen, bevorzugt ein oder mehrere Elemente aus der Gruppe bestehend aus Bor, Gallium, Silizium, Germanium, Zinn und Blei, besonders bevorzugt Zinn.

In einer bevorzugten Ausführungsform enthält der Dehydrierungskatalysator mindestens ein Element der VIII. Nebengruppe, mindestens ein Element der I. und/oder II. Hauptgruppe, mindestens ein Element der III. und/oder IV. Hauptgruppe und mindestens ein Element der III. Nebengruppe einschließlich der Lanthaniden und Actiniden.

Beispielsweise können erfindungsgemäß alle Dehydrierkatalysatoren eingesetzt werden, die in den WO 99/46039, US 4,788,371, EP-A 705 136, WO 99/29420, US 5,220,091, US 5,430,220, US 5,877,369, EP 0 117 146, DE-A 199 37 106, DE-A 199 37 105 und DE-A 199 37 107 offenbart werden. Besonders bevorzugte Katalysatoren für die vorstehend beschriebenen Varianten der autothermen n-Butan-Dehydrierung sind die Katalysatoren gemäß den Beispielen 1, 2, 3 und 4 der DE-A 199 37 107.

Die n-Butan-Dehydrierung wird bevorzugt in Gegenwart von Wasserdampf durchgeführt. Der zugesetzte Wasserdampf dient als Wärmeträger und unterstützt die Vergasung von organischen Ablagerungen auf den Katalysatoren, wodurch der Verkokung der Katalysatoren entgegengewirkt und die Standzeit der Katalysatoren erhöht wird. Dabei werden die organischen Ablagerungen in Kohlenmonoxid, Kohlendioxid und gegebenenfalls Wasser umgewandelt.

Die nicht-oxidative, katalytische n-Butan-Dehydrierung kann auch mit Kreisgasfahrweise betrieben werde, wodurch höhere Umsätze erzielt werden.

Der Dehydrierungskatalysator kann in an sich bekannter Weise regeneriert werden. So kann dem Reaktionsgasgemisch Wasserdampf zugesetzt werden oder von Zeit zu Zeit ein Sauerstoff enthaltendes Gas bei erhöhter Temperatur über die Katalysatorschüttung geleitet werden und der abgeschiedene Kohlenstoff abgebrannt werden. Durch die Verdünnung mit Wasserdampf wird das Gleichgewicht zu den Produkten der Dehydrierung verschoben. Gegebenenfalls wird der Katalysator nach der Regenerierung mit einem wasserstoffhaltigen Gas reduziert.

Bei der nicht-oxidativen katalytischen n-Butan-Dehydrierung wird ein Gasgemisch erhalten, das neben Butadien 1-Buten, 2-Buten und nicht umgesetztem n-Butan Nebenbestandteile enthält. Übliche Nebenbestandteile sind Wasserstoff, Wasserdampf, Stickstoff, CO und CO₂ Methan, Ethan, Ethen, Propan und Propen. Die Zusammensetzung des die erste Dehydrierzone verlassenden Gasgemischs kann abhängig von der Fahrweise der Dehydrierung stark variieren. So weist bei Durchführung der bevorzugten autothermen Dehydrierung unter Einspeisung von Sauerstoff und zusätzlichem Wasserstoff das Produktgasgemisch einen vergleichsweise hohen Gehalt an Wasserdampf und Kohlenstoffoxiden auf. Bei Fahrweisen ohne Einspeisung von Sauerstoff weist das Produktgasgemisch der nicht-oxidativen Dehydrierung einen vergleichsweise hohen Gehalt an Wasserstoff auf.

Der Produktgasstrom der nicht-oxidativen autothermen n-Butan-Dehydrierung enthält typischerweise 0,1 bis 15 Vol.-% Butadien, 1 bis 15 Vol.-% 1-Buten, 1 bis 25 Vol.-% 2-Buten (cis/trans-2-Buten), 20 bis 70 Vol.-% n-Butan, 1 bis 70 Vol.-% Wasserdampf, 0 bis 10 Vol.-% leichtsiedende Kohlenwasserstoffe (Methan, Ethan, Ethen, Propan und Propen), 0,1 bis 40 Vol.-% Wasserstoff, 0 bis 10 Vol.-% Stickstoff und 0 bis 5 Vol.-% Kohlenstoffoxide.

Der die erste Dehydrierzone verlassende Produktgasstrom b kann in zwei Teilströme aufgetrennt werden, wobei nur einer der beiden Teilströme den weiteren Verfahrensteilen C bis H unterworfen wird und der zweite Teilstrom in die erste Dehydrierzone zurückgeführt wird. Eine entsprechende Verfahrensweise ist in DE-A 102 11 275 beschrieben. Es kann jedoch auch der gesamte Produktgasstrom b der nicht-oxidativen katalytischen n-Butan-Dehydrierung den weiteren Verfahrensteilen C bis H unterworfen werden.

Der nicht-oxidativen katalytischen Dehydrierung wird erfindungsgemäß eine oxidative Dehydrierung (Oxidehydrierung) als Verfahrensteil C nachgeschaltet. Dabei werden im Wesentlichen 1-Buten und 2-Buten zu 1,3-Butadien dehydriert, wobei 1-Buten im Allgemeinen nahezu vollständig abreagiert.

Diese kann grundsätzlich in allen aus dem Stand der Technik bekannten Reaktortypen und Fahrweisen durchgeführt werden, wie beispielsweise im Wirbelbett, im Hordenofen im Festbettrohr- oder -rohrbündelreaktor oder im Plattenwärmetauscherreaktor. Zur Durchführung der oxidativen Dehydrierung wird ein Gasgemisch benötigt, welches ein molares Sauerstoff : n-Butene-Verhältnis von mindestens 0,5 aufweist. Bevorzugt wird bei einem Sauerstoff : n-Butene-Verhältnis von 0,55 bis 50 gearbeitet. Zur Einstellung dieses Wertes wird in der Regel das aus der nicht-oxidativen katalytischen Dehydrierung stammende Produktgasgemisch mit reinem Sauerstoff oder einem sauerstoffhaltigem Gas vermischt. Das sauerstoffhaltige Gas enthält wie im Falle der ersten (autothermen) Dehydrierstufe B) überwiegend Sauerstoff, mindestens 75 Vol.%, bevorzugt mindestens 90 Vol.%, um den Inertgasanteil im Produktgasstrom der Oxidehydrierung zu minimieren. Bevorzugt ist technisch reiner Sauerstoff. Das erhaltene sauerstoffhaltige Gasgemisch wird dann der Oxidehydrierung zugeführt.

Die für die Oxidehydrierung besonders geeigneten Katalysatoren basieren im Allgemeinen auf einem Mo-Bi-O-haltigen Multimetalloxidsystem, das in der Regel zusätzlich Eisen enthält. Im Allgemeinen enthält das Katalysatorsystem noch weitere zusätzliche Komponenten aus der 1. bis 15. Gruppe des Periodensystems, wie beispielsweise Kalium, Magnesium, Zirkon, Chrom, Nickel, Cobalt, Cadmium, Zinn, Blei, Germanium, Lanthan, Mangan, Wolfram, Phosphor, Cer, Aluminium oder Silizium.

Geeignete Katalysatoren und deren Herstellung sind beispielsweise beschrieben in US 4,423,281 (Mo₁₂BiNi₈Pb_{0,5}Cr₃K_{0,2}Oₓ und Mo₁₂Bi_{b}Ni₇Al₃Cr_{0,5}K_{0,5}Oₓ), US 4,336,409 (Mo₁₂BiNi₆Cd₂Cr₃P_{0,5}Oₓ), DE-A 26 00 128 (Mo₁₂BiNi_{0,5}Cr₃P_{0,5}Mg_{7,5}K_{0,1}Oₓ + SiO₂) und DE-A 24 40 329 (Mo₁₂BiCo_{4,5}Ni_{2,5}Cr₃P_{0,5}K_{0,1}Oₓ).

Die Stöchiometrie der Aktivmasse einer Vielzahl der für die Oxidehydrierung geeigneten Multimetalloxidkatalysatoren lässt sich unter die allgemeine Formel (I)

Mo₁₂BiₐFe_{b}Co_{c}Ni_{d}CrₑX¹_{f}k_{g}Oₓ (I),

in der die Variablen nachfolgende Bedeutung aufweisen:
- X¹ =: W, Sn, Mn, La, Ce, Ge, Ti, Zr, Hf, Nb, P, Si, Sb, Al, Cd und/oder Mg;
- a =: 0,5 bis 5, vorzugsweise 0,5 bis 2;
- b =: 0 bis 5, vorzugsweise 2 bis 4;
- c =: 0 bis 10, vorzugsweise 3 bis 10;
- d =: 0 bis 10;
- e =: 0 bis 10, vorzugsweise 0,1 bis 4;
- f =: 0 bis 5, vorzugsweise 0,1 bis 2;
- g =: 0 bis 2, vorzugsweise 0,01 bis 1; und
- x =: eine Zahl, die durch die Wertigkeit und Häufigkeit der von Sauerstoff verschiedenen Elemente in (I) bestimmt wird;
subsumieren.

Bevorzugt setzt man beim erfindungsgemäßen Verfahren für die Oxidehydrierung ein Mo-Bi-Fe-O-haltiges Multimetalloxidsystem ein, wobei ein Mo-Bi-Fe-Cr-O- oder Mo-Bi-Fe-Zr-O-haltiges Multimetalloxidsystem besonders bevorzugt ist. Bevorzugte Systeme sind beispielsweise beschrieben in US 4,547,615 (Mo₁₂BiFe_{0,1}Ni₈ZrCr₃K_{0.2}Oₓ und Mo₁₂BiFe_{0,1}Ni₈AlCr₃K_{0,2}Oₓ), US 4,424,141 (Mo₁₂BiFe₃Co_{4,5}Ni_{2,5}P_{0,5}K₀,₁Oₓ + SiO₂), DE-A 25 30 959 (Mo₁₂BiFe₃Co_{4,5}Ni_{2,5}Cr_{0,5}K_{0,1}Oₓ, Mo_{13,75}BiFe₃Co_{4,5}Ni_{2,5}Ge_{0,5}K_{0,8}Oₓ, Mo₁₂BiFe₃Co_{4,5}Ni_{2,5}Mn_{0,5}K_{0,1}Oₓ und Mo₁₂BiFe₃Co_{4,5}Ni_{2,5}La_{0,5}K_{0,1}Oₓ), US 3,911,039 (Mo₁₂BiFe₃Co_{4,5}Ni_{2,5}Sn_{0,5}K_{0,1}Oₓ), DE-A 25 30 959 und DE-A 24 47 825 (Mo₁₂BiFe₃Co_{4,5}Ni_{2,5}W_{0,5}K_{0,1}Oₓ). Herstellung und Charakterisierung der genannten Katalysatoren sind ausführlich in den zitierten Schriften beschrieben.

Der Katalysator zur Oxidehydrierung wird im Allgemeinen als Formkörper mit einer mittleren Größe von über 2 mm eingesetzt. Aufgrund des zu beachtenden Druckverlustes während der Ausübung des Verfahrens sind kleinere Formkörper in der Regel ungeeignet. Als geeignete Formkörper seien beispielsweise genannt Tabletten, Zylinder, Hohlzylinder, Ringe, Kugeln, Stränge, Wagenräder oder Extrudate. Besondere Formen, wie beispielsweise "Trilobes" und "Tristars" (siehe EP-A-0 593 646) oder Formkörper mit mindestens einer Einkerbung an der Außenseite (siehe US 5,168,090) sind ebenfalls möglich.

Im Allgemeinen kann der verwendete Katalysator als sogenannter Vollkatalysator eingesetzt werden. In diesem Fall besteht der gesamte Katalysatorformkörper aus der Aktivmasse, inklusive eventueller Hilfsmittel, wie etwa Graphit oder Porenbildner, sowie weiterer Komponenten. Insbesondere hat es sich als günstig erwiesen, den für die Oxidehydrierung der n-Butene zu Butadien bevorzugt eingesetzten Mo-Bi-Fe-O-haltigen Katalysator als Vollkatalysator einzusetzen. Des Weiteren ist es möglich, die Aktivmassen der Katalysatoren auf einen Träger, beispielsweise einen anorganischen, oxidischen Formkörper, aufzubringen. Derartige Katalysatoren werden in der Regel als Schalenkatalysatoren bezeichnet.

Die Oxidehydrierung wird im Allgemeinen bei einer Temperatur von 220 bis 490 °C und bevorzugt von 250 bis 450 °C durchgeführt. Man wählt einen Reaktoreingangsdruck, der ausreicht, die in der Anlage und der nachfolgenden Aufarbeitung vorhandenen Strömungswiderstände zu überwinden. Dieser Reaktoreingangsdruck liegt in der Regel bei 0,005 bis 1 MPa Überdruck, bevorzugt 0,01 bis 0,5 MPa Überdruck. Naturgemäß fällt der im Eingangsbereich des Reaktors angewendete Gasdruck weitgehend über die gesamte Schüttung aus Katalysator ab.

Durch die Kopplung der nicht-oxidativen katalytischen, bevorzugt autothermen Dehydrierung mit der oxidativen Dehydrierung der gebildeten n-Butene wird eine sehr viel höhere Ausbeute an Butadien, bezogen auf eingesetztes n-Butan, erhalten. Ferner kann die nicht-oxidative Dehydrierung schonender betrieben werden. Vergleichbare Butadien-Ausbeuten wären mit einer ausschließlich nicht-oxidativen Dehydrierung nur zum Preis deutlich niedrigerer Selektivitäten zu erreichen. Mit einer ausschließlich oxidativen Dehydrierung werden nur geringe n-Butan-Umsätze erzielt.

Der die oxidative Dehydrierung verlassende Produktgasstrom c enthält neben Butadien und nicht umgesetztem n-Butan noch Wasserstoff, Kohlendioxid und Wasserdampf. Als Nebenbestandteile kann er noch Sauerstoff, Stickstoff, Methan, Ethan, Ethen, Propan und Propen sowie sauerstoffhaltige Kohlenwasserstoffe, sogenannte Oxygenate, enthalten. Im Allgemeinen enthält er praktisch kein 1-Buten mehr und nur noch geringe Anteile an 2-Buten.

Im Allgemeinen weist der die oxidative Dehydrierung verlassende Produktgasstrom c folgende typische Zusammensetzung auf: 10 bis 40 Vol.-% Butadien, 15 bis 79,9 Vol.-% n-Butan, 0 bis 10 Vol.-% 2-Buten, 0 bis 2 Vol.-% 1-Buten, 10 bis 70 Vol.-% Wasserdampf, 0 bis 10 Vol.-% leichtsiedende Kohlenwasserstoffe (Methan, Ethan, Ethen, Propan und Propen), 0,1 bis 15 Vol.-% Wasserstoff, 0 bis 10 Vol.-% Stickstoff, 0 bis 15 Vol.-% Kohlendioxid und 0 bis 7 Vol.-% Oxygenate auf. Oxygenate können beispielsweise Furan, Essigsäure, Maleinsäureanhydrid, Maleinsäure, Propionsäure, Acetaldehyd, Acrolein, Formaldehyd, Ameisensäure und Butyraldehyd sein. Daneben können in Spuren Acetylen, Propin und 1,2-Butadien enthalten sein.

Der Produktgasstrom c kann noch geringe Mengen Sauerstoff enthalten. Enthält der Produktgasstrom c mehr als nur geringfügige Spuren Sauerstoff, so wird vorzugsweise eine katalytische Verbrennungsstufe durchgeführt, in der Sauerstoff mit dem in dem Gasstrom c enthaltenen Wasserstoff in Gegenwart eines Katalysators umgesetzt wird. Hierdurch wird eine Verringerung des Sauerstoffgehalts bis auf geringe Spuren erreicht.

Ein geeigneter Katalysator für die Oxidation von Wasserstoff enthält, geträgert auf α-Aluminiumoxid, 0,01 bis 0,1 Gew.-% Platin und 0,01 bis 0,1 Gew.-% Zinn bezogen auf das Gesamtgewicht des Katalysators. Platin und Zinn werden vorteilhaft in einem Gewichtsverhältnis von 1:4 bis 1:0,2 eingesetzt, bevorzugt in einem Verhältnis von 1:2 bis 1:0,5, insbesondere in einem Verhältnis von annährend 1:1. Vorteilhaft enthält der Katalysator 0,05 bis 0,09 Gew.-% Platin und 0,05 bis 0,09 Gew.-% Zinn bezogen auf das Gesamtgewicht des Katalysators. Neben Platin und Zinn können gegebenenfalls Alkali- und/oder Erdalkalimetallverbindungen in Mengen kleiner 2 Gew.-%, insbesondere kleiner 0,5 Gew.-% verwendet werden. Besonders bevorzugt enthält der Aluminiumoxid-Katalysator ausschließlich Platin und Zinn. Der Katalysatorträger aus α-Aluminiumoxid weist vorteilhaft eine BET-Oberfläche von 0,5 bis 15 m²/g auf, bevorzugt 2 bis 14 m²/g, insbesondere 7 bis 11 m²/g. Als Träger wird bevorzugt ein Formkörper eingesetzt. Bevorzugte Geometrien sind beispielsweise Tabletten, Ringtabletten, Kugeln, Zylinder, Sternstränge oder zahnradförmige Stränge mit Durchmessern von 1 bis 10 mm, bevorzugt 2 bis 6 mm. Besonders bevorzugt sind Kugeln oder Zylinder, insbesondere Zylinder.

In einer Verfahrensstufe D) wird der Gasstrom c in mindestens einer ersten Kompressionsstufe komprimiert und anschließend abgekühlt, wobei mindestens ein Kondensatstrom d1 enthaltend Wasser auskondensiert und ein Gasstrom d2 enthaltend n-Butan, Butadien, Wasserstoff, Kohlendioxid und Wasserdampf verbleibt.

Die Kompression kann ein- oder mehrstufig erfolgen. Im Allgemeinen wird insgesamt von einem Druck im Bereich von 1,0 bis 4,0 bar (absolut) auf einen Druck im Bereich von 3,5 bis 8,0 bar komprimiert. Nach jeder Kompressionsstufe folgt eine Abkühlstufe, in der der Gasstrom auf eine Temperatur im Bereich von im Allgemeinen 15 bis 60 °C abgekühlt wird. Der Kondensatstrom d1 kann somit bei mehrstufiger Kompression auch mehrere Ströme umfassen.

Der Gasstrom d2 besteht im Allgemeinen im Wesentlichen aus C₄-Kohlenwasserstoffen (im Wesentlichen n-Butan und Butadien), Wasserstoff, Kohlendioxid und Wasserdampf. Die genannten Komponenten machen im Allgemeinen insgesamt mindestens 80 Gew.-%, vorzugsweise mindestens 90 Gew.-% des Gasstroms d2 aus. Daneben kann der Strom d2 noch Leichtsieder, Sauerstoff und Inertgase (Stickstoff) als weitere Nebenkomponenten enthalten. Der Abwasserstrom d1 besteht im Allgemeinen zu mindestens 80 Gew.%, vorzugsweise zu mindestens 90 Gew.-% aus Wasser und enthält daneben in geringem Umfang Leichtsieder, C₄-Kohlenwasserstoffe, Oxygenate und Kohlendioxid.

In einer Verfahrenstufe E) wird der Gasstrom d2 in mindestens einer weiteren Kompressionsstufe weiter komprimiert und anschließend abgekühlt, wobei mindestens ein Kondensatstrom e1 enthaltend n-Butan, Butadien und Wasser auskondensiert und ein Gasstrom e2 enthaltend n-Butan, Butadien, Wasserstoff und Kohlendioxid verbleibt.

Die Kompression kann wiederum ein- oder mehrstufig erfolgen. Im Allgemeinen wird insgesamt von einem Druck im Bereich von 3,5 bis 8,0 bar auf einen Druck im Bereich von 12 bis 40 bar komprimiert. Nach jeder Kompressionsstufe folgt eine Abkühlstufe, in der der Gasstrom auf eine Temperatur im Bereich von im Allgemeinen 15 bis 60 °C abgekühlt wird. Der Kondensatstrom e1 kann somit bei mehrstufiger Kompression auch mehrere Ströme umfassen.

Der Gasstrom e2 besteht im Allgemeinen im Wesentlichen aus C₄-Kohlenwasserstoffe (im Wesentlichen n-Butan und Butadien), Wasserstoff und Kohlendioxid, das heißt die genannten Komponenten machen insgesamt mindestens 80 Gew.-%, vorzugsweise mindestens 90 Gew.-% des Gasstroms e2 aus. Daneben kann er noch in geringen Mengen Leichtsieder, Sauerstoff und Inertgase (Stickstoff) als weitere Nebenkomponenten enthalten. Auch Wasserdampf kann noch in geringen Mengen enthalten sein. Der Kondensatstrom e1 besteht im Allgemeinen zu mindestens 50 Gew.-%, vorzugsweise zu mindestens 80 Gew.-% aus C₄-Kohlenwasserstoffen (im Wesentlichen n-Butan und Butadien, daneben gegebenenfalls noch Butene) und enthält daneben noch Wasser, Leichtsieder, Oxygenate und Kohlendioxid.

Geeignete Verdichter sind beispielsweise Turbo-, Drehkolben- und Hubkolbenverdichter. Die Verdichter können beispielsweise mit einem Elektromotor, einem Expander oder einer Gas- oder Dampfturbine angetrieben werden. Typische Verdichtungsverhältnisse (Austrittsdruck : Eintrittsdruck) pro Verdichterstufe liegen je nach Bauart zwischen 1,5 und 3,0.

Die Abkühlung des verdichteten Gases erfolgt mit Wärmetauschern, die beispielsweise als Rohrbündel-, Spiral- oder Plattenwärmetauscher ausgeführt sein können. Als Kühlmittel kommen in den Wärmetauschern dabei Kühlwasser oder Wärmeträgeröle zum Einsatz. Daneben wird bevorzugt Luftkühlung unter Einsatz von Gebläsen eingesetzt.

In einem Verfahrenschritt F) wird der Gasstrom e2 abgekühlt, wobei ein Kondensatstrom f1 enthaltend n-Butan und Butadien und ein Abgasstrom f2 enthaltend Kohlendioxid und Wasserstoff erhalten werden. Im Allgemeinen wird der Gastrom e2 auf eine Temperatur im Bereich von -30 bis +20 °C abgekühlt. Der Kondensatstrom f1 enthält überwiegend C₄-Kohlenwasserstoffe (im Wesentlichen n-Butan und Butadien), im Allgemeinen zu mindestens 60 Gew.-%, vorzugsweise zu mindestens 70 Gew.-%, und enthält daneben noch Kohlendioxid. Daneben kann er noch Leichtsieder sowie geringe Mengen Wasser enthalten.

Der Kondensatstrom f1 kann beispielsweise in Oberflächenkondensatoren auskondensiert werden. In diesen kommt der Gasstrom e2 mit Rohren, welche von einem Kühlmedium durchströmt werden, in Kontakt. Als Kühlmittel kommen beispielsweise Wasser, Luft und Kühlsole in Betracht. Auch Einspritzkondensatoren, bei denen das Kühlmittel, vorzugsweise Wasser, direkt in den Gasstrom, der die aus zu kondensierenden Komponenten enthält, eingespritzt wird, sind möglich.

In einem Verfahrensschritt G) wird aus dem oder den Kondensatströmen e1 und gegebenenfalls dem Kondensatstrom f1 durch Phasentrennung Wasser abgetrennt, wobei ein C₄-Kohlenwasserstoffstroms g1 enthaltend n-Butan und Butadien und mindestens ein Abwasserstrom g2 erhalten werden.

Hierzu werden die Kondensatströme getrennt oder vereinigt einem bzw. mehreren Phasentrennapparaten zugeführt. Es werden ein vereinigter oder mehrere getrennte C₄-Kohlenwasserstoffströme g1 erhalten. Der oder die Kohlenwasserstoffströme enthalten überwiegend C₄-Kohlenwasserstoffe (im Wesentlichen n-Butan und Butadien), im Allgemeinen zu mindestens 80 Gew.-%, vorzugsweise zu mindestens 85 Gew.-%, und können daneben noch Kohlendioxid und Leichtsieder enthalten. Der Abwasserstrom g2 enthält überwiegend Wasser, im Allgemeinen zu mindestens 70 Gew.-%, und enthält daneben im Allgemeinen noch Kohlendioxid. Daneben können noch Kohlenwasserstoffe (Leichtsieder und C₄-Kohlenwasserstoffe) in geringen Mengen, vorzugsweise bis zu maximal 10 Gew.-%, enthalten sein. Der Abgasstrom g2 enthält im Allgemeinen überwiegend Kohlendioxid, daneben Wasserstoff, in geringem Umfang C₄-Kohlenwasserstoffe sowie gegebenenfalls Leichtsieder und Inertgase (Stickstoff).

Zur Abtrennung des im Abgasstrom g2 enthaltenen Wasserstoffs kann dieser, gegebenenfalls nach erfolgter Kühlung, beispielsweise in einem indirekten Wärmetauscher, über eine in der Regel als Rohr ausgebildete Membran geleitet werden, die lediglich für molekularen Wasserstoff durchlässig ist. Alternativ kann der Wasserstoff auch mittels Druckwechselabsorption (PSA) abgetrennt werden. Der so abgetrennte molekulare Wasserstoff kann bei Bedarf zumindest teilweise in der Dehydrierung eingesetzt oder aber einer sonstigen Verwertung zugeführt werden, beispielsweise zur Erzeugung elektrischer Energie in Brennstoffzellen eingesetzt werden.

In einem Verfahrensschritt H) wird der C₄-Kohlenwasserstoffstrom g1 in einen n-Butan enthaltenden Rückführstrom h1 und einen im Wesentlichen aus Butadien bestehenden Produktstrom h2 aufgetrennt und wird der n-Butan enthaltenden Rückführstrom h1 in die erste Dehydrierzone zurückgeführt.

Die Auftrennung ist vorzugsweise als Extraktivdestillation ausgestaltet. Die Extraktivdestillation kann beispielsweise wie in Erdöl und Kohle - Erdgas - Petrochemie Band 34 (8), Seiten 343 - 346 oder Ullmanns Enzyklopädie der Technischen Chemie, Band 9, 4. Auflage 1975, Seiten 1 bis 18 beschrieben durchgeführt werden.

Hierzu wird der C₄-Produktgasstrom g1 mit einem Extraktionsmittel, vorzugsweise einem N-Methylpyrrolidon (NMP)/Wasser-Gemisch, in einer Extraktionszone in Kontakt gebracht. Die Extraktionszone ist im Allgemeinen in Form einer Waschkolonne ausgeführt, welche Böden, Füllkörper oder Packungen als Einbauten enthält. Diese weist im Allgemeinen 30 bis 70 theoretische Böden auf, damit eine hinreichend gute Trennwirkung erzielt wird. Vorzugsweise weist die Waschkolonne im Kolonnenkopf eine Rückwaschzone auf. Diese Rückwaschzone dient zur Rückgewinnung des in der Gasphase enthaltenen Extraktionsmittels mittels flüssigem Kohlenwasserstoffrücklauf, wozu die Kopffraktion zuvor kondensiert wird. Typische Temperaturen am Kopf der Kolonne liegen zwischen 30 und 60 °C. Das Massenverhältnis Extraktionsmittel zu C₄-Produktgasstrom g1 im Zulauf der Extraktionszone beträgt im allgemeinen 10 : 1 bis 20 : 1.

Geeignete Extraktionsmittel sind Butyrolacton, Nitrile wie Acetonitril, Propionitril, Methoxypropionitril, Ketone wie Aceton, Furfural, N-alkylsubstituierte niedere aliphatische Säureamide wie Dimethylformamid, Diethylformamid, Dimethylacetamid, Diethylacetamid, N-Formylmorpholin, N-alkylsubstituierte cyclische Säureamide (Lactame) wie N-Alkylpyrrolidone, insbesondere N-Methylpyrrolidon (NMP). Im Allgemeinen werden alkylsubstituierte niedere aliphatische Säureamide oder N-alkylsubstituierte cyclische Säureamide verwendet. Besonders vorteilhaft sind Dimethylformamid, Acetonitril, Furfural und insbesondere NMP.

Es können jedoch auch Mischungen dieser Extraktionsmittel untereinander, z. B. von NMP und Acetonitril, Mischungen dieser Extraktionsmittel mit Co-Lösungsmitteln und/oder tert.-Butylether, z. B. Methyl-tert.-butylether, Ethyl-tert.-butylether, Propyl-tert.-butylether, n- oder iso-Butyl-tert.-butylether eingesetzt werden. Besonders geeignet ist NMP, bevorzugt in wässriger Lösung, vorzugsweise mit 0 bis 20 Gew.-% Wasser, besonders bevorzugt mit 7 bis 10 Gew.-% Wasser, insbesondere mit 8,3 Gew.-% Wasser.

In der Extraktivdestillationskolonne wird ein gasförmiger, im Wesentlichen aus n-Butan bestehender Strom h1, der im Allgemeinen über den Kolonnenkopf abgezogen wird, und eine Butadien enthaltende Extraktionslösung gebildet. Als Seitenabzugsstrom wird ein Gemisch aus Extraktionsmittel und Butadien gewonnen. Aus diesem Gemisch kann Butadien nachfolgend als Reinprodukt gewonnen werden. Als Sumpfabzugsstrom wird das Extraktionsmittel, welches noch Butadien und gegebenenfalls Nebenkomponenten (Verunreinigungen) enthält, gewonnen. Der Sumpfabzugsstrom wird, gegebenenfalls nach Durchführung weiterer Aufreinigungsschritten, wieder in die Extraktivdestillation zurückgeführt.

Beispielsweise kann die Extraktivdestillation, Isolierung des Rein-Butadiens und Aufreinigung des Extraktionsmittels wie folgt durchgeführt werden: Der Seitenabzugsstrom der Extraktivdestillationskolonne aus Extraktionsmittel und Butadien, der noch Verunreinigungen enthält (Acetylen, Propin, 1,2-Butadien), wird in eine Waschkolonne eingespeist, welche mit frischem Extraktionsmittel beschickt wird. Am Kolonnenkopf der Waschkolonne wird Roh-Butadien, welches beispielsweise 98 Gew.-% Butadien enthält, abgezogen. Der Sumpfabzugsstrom ist mit Acetylen angereichert und wird in die Extraktivdestillation zurückgeführt. Das Roh-Butadien kann als Verunreinigungen Propin und 1,2-Butadien enthalten. Zur Entfernung dieser Verunreinigungen wird das Roh-Butadien einer ersten Reindestillationskolonne zugeführt und über Kopf ein mit Propin angereicherter Butadienstrom abgetrennt. Der Sumpfabzugsstrom, der im Wesentlichen Propin-frei ist, aber noch Spuren von 1,2-Butadien enthält, wird in eine zweite Reindestillationskolonne eingespeist, in der ein im Wesentlichen 1,2-Butadien-freier Rein-Butadienstrom mit einer Reinheit von beispielsweise mindestens 99,6 Gew.-% als Kopfabzugsstrom oder Seitenabzugsstrom im Verstärkungsteil der Kolonne und ein mit 1,2-Butadien angereicherter Sumpfabzugsstrom erhalten werden.

Zur Aufreinigung des Extraktionsmittels wird ein Teil des Extraktionsmittels als Sumpfabzugsstrom aus der Extraktivdestillationskolonne ausgeschleust und wie folgt regeneriert: Die Extraktionslösung wird in eine Desorptionszone mit gegenüber der Extraktionszone vermindertem Druck und/oder erhöhter Temperatur überführt, wobei aus der Extraktionslösung Butadien und vorhandene Acetylen-Spuren desorbiert werden. Die Desorptionszone kann beispielsweise in Form einer Waschkolonne ausgeführt sein, die 5 bis 15, bevorzugt 8 bis 10 theoretische Stufen und eine Rückwaschzone mit beispielsweise 4 theoretischen Stufen aufweist. Diese Rückwaschzone dient zur Rückgewinnung des in der Gasphase enthaltenen Extraktionsmittels mittels flüssigem Kohlenwasserstoffrücklaufes, wozu die Kopffraktion zuvor kondensiert wird. Als Einbauten sind Packungen, Böden oder Füllkörper vorgesehen. Der Druck am Kolonnenkopf beträgt beispielsweise 1,5 bar. Die Temperatur im Kolonnensumpf liegt beispielsweise bei 130 bis 150 °C. Am Kolonnensumpf wird ein im Wesentlichen Acetylen-freies Extraktionsmittel erhalten, welches in die Extraktivdestillationskolonne zurückgeführt wird.

Der Wertproduktstrom h2, wie er beispielsweise als Kopfabzugsstrom der zweiten Reindestillationskolonne erhalten wird, kann bis zu 100 Vol.-% Butadien enthalten.

Der im Wesentlichen aus n-Butan bestehende Strom h1 enthält im Allgemeinen mindestens 70 Gew.-% n-Butan und daneben als weitere Bestandteile noch Kohlendioxid, gegebenenfalls Butadien, gegebenenfalls Butene und Leichtsieder.

### Beispiel

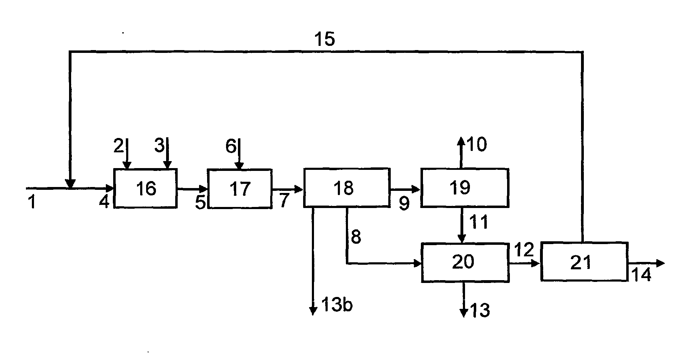

Mittels eines Simulationsprogramms wurde die in der Figur dargestellte Verfahrensvariante simuliert. Ein n-Butan enthaltender Einsatzgasstrom (4), welcher durch Vereinigung eines Frischgasstroms (1) und eines Rückführstroms (15) erhalten wird, wird der ersten, autotherm betriebenen, nicht-oxidativen katalytischen n-Butan-Dehydrierungsstufe (BDH) (16) zugeführt. Zur Bereitstellung der für die endotherme Dehydrierung notwendigen Wärme wird selektiv ein Teil des Wasserstoffs verbrannt, der bei der Dehydrierung gebildet wird. Zu diesem Zweck wird Verbrennungsluft als Strom (2) zugeführt. Um einer Verkokung des Katalysators entgegenzuwirken und die Standzeit des Katalysators zu verlängern wird außerdem Wasserdampf (3) zugegeben. Es wird ein Dehydriergasgemisch (5) erhalten, das nach Austritt aus der autothermen Dehydrierstufe (16) abgekühlt und der zweiten, oxidativen n-Butan-Dehydrierstufe (ODH) (17) zugeführt wird. Der ODH (17) wird ferner ein Sauerstoffstrom (6) zugeführt. Für BDH und ODH wurden basierend auf experimentellen Ergebnissen die in Tabelle 1 wiedergegebenen Umsatzgrade bzw. Selektivitäten angenommen.

**Tabelle 1**

| Reaktionsstufe | Umsatz [%] | Selektivität [%] |
|---|---|---|
| Autotherme Dehydrierung (BDH) | 49,5 | 97,9 |
| | (n-Butan) | (zu Butenen/Butadien) |
| Oxidative Dehydrierung (ODH) | 100,0 (1-Buten) | 95,0 |
| | 92,7(2-Buten) | (zu Butadien) |

Das Austrittsgas der Oxidehydrierung (7), weiches unter einem Druck von 2,4 bar (absolut) steht, wird in mehreren Verdichtern (18) mehrstufig mit Zwischenkühlung auf einen Druck von 30,1 bar verdichtet und das verdichtete Gas (9) in einem Kondensator (19) auf eine Temperatur von 5°C abgekühlt. Das nicht-kondensierte Abgas (10) ist wasserstoffreich und wird entweder einer Verbrennung oder einer stofflichen Verwertung (Druckwechselabsorption, Membranabtrennung des Wasserstoffs) zugeführt. Das nach der zweiten Verdichterstufe und Zwischenkühlung bei einem Druck von 12 bar und einer Temperatur von 55 °C anfallende Kondensat (8) und das nach der dritten Verdichterstufe bei dem Enddruck von 30,1 bar und der Temperatur von 5 °C anfallende Kondensat (11) werden in einem Phasenscheider (20) in eine wässrige Phase (Abwasserstrom 13) und eine organische Phase (12) getrennt. Das nach der ersten Verdichterstufe und Zwischenkühlung bei einem Druck von 5 bar und einer Temperatur von 55 °C gewonnene Kondensat (13b) kann direkt dem Abwasser zugeführt werden. Abschließend erfolgt eine Butadien-Extraktion in einer Extraktivdestillation unter Verwendung von NMP als Lösungsmittel und Abtrennung des Butadiens (14). (21) bezeichnet hier die Gesamtheit aus Extraktionskolonne, Waschkolonne und Butadien-Reindestillationskolonnen (21). Der nach der Butadien-Extraktion verbleibende n-Butan reiche Strom (15) wird mit dem frischen n-Butan-Einsatzgasstrom (1) vereinigt und in die erste Dehydrierstufe zurückgeführt.

Die Ergebnisse der Simulationsrechnung sind in Tabelle 2 wiedergegeben. Die Zusammensetzung der Stoffströme (1) bis (15) ist in Gewichtsanteilen angegeben.

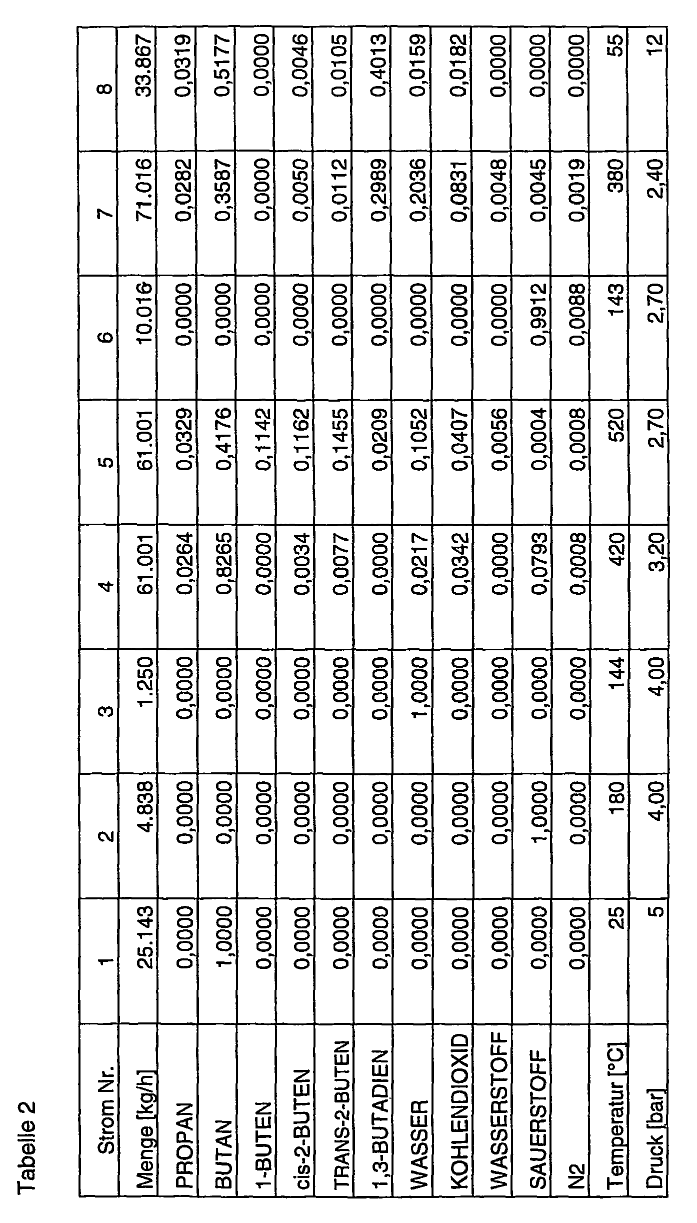

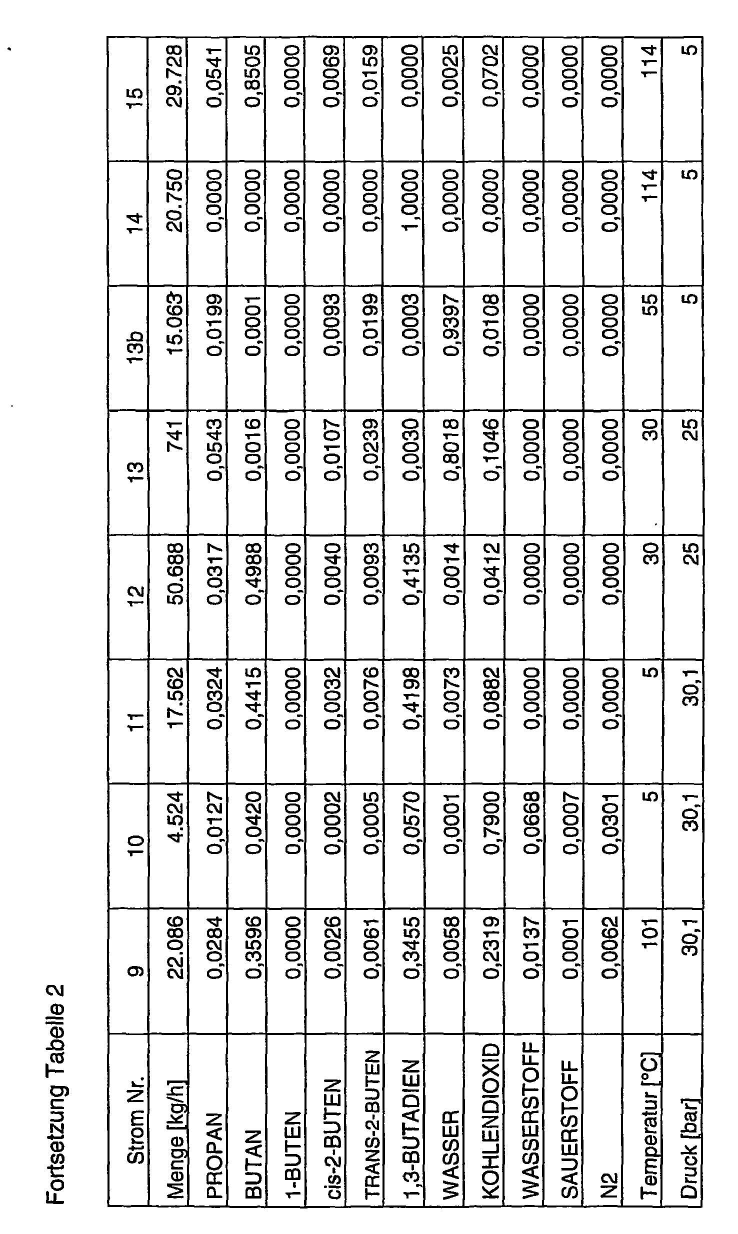

## Patentansprüche

1. Verfahren zur Herstellung von Butadien aus n-Butan mit den Schritten
A) Bereitstellung eines n-Butan enthaltenden Einsatzgasstroms a;
B) Einspeisung des n-Butan enthaltenden Einsatzgasstroms a in mindestens eine erste Dehydrierzone und nicht-oxidative, katalytische Dehydrierung von n-Butan, wobei ein Gasstrom b enthaltend n-Butan, 1-Buten, 2-Buten, Butadien, Wasserstoff, gegebenenfalls Kohlendioxid und gegebenenfalls Wasserdampf erhalten wird;
C) Einspeisung des Gasstroms b und eines sauerstoffhaltigen Gases mit einem Sauerstoffgehalt von mindestens 75 Vol.-% in mindestens eine zweite Dehydrierzone und oxidative Dehydrierung von 1-Buten und 2-Buten, wobei ein Gasstrom c enthaltend n-Butan, Butadien, Wasserstoff, Kohlendioxid und Wasserdampf erhalten wird,
D) Kompression in mindestens einer ersten Kompressionsstufe und Abkühlung des Gasstroms c, wobei mindestens ein Kondensatstrom d1 enthaltend Wasser und ein Gasstrom d2 enthaltend n-Butan, Butadien, Wasserstoff, Kohlendioxid und Wasserdampf erhalten wird,
E) Kompression in mindestens einer weiteren Kompressionsstufe und Abkühlung des Gasstroms d2, wobei mindestens ein Kondensatstrom e1 enthaltend n-Butan, Butadien und Wasser und ein Gasstrom e2 enthaltend n-Butan, Butadien, Wasserstoff und Kohlendioxid erhalten werden,
F) Abkühlung des Gasstroms e2, wobei ein Kondensatstrom f1 enthaltend n-Butan und Butadien und ein Abgasstrom f2 enthaltend Kohlendioxid und Wasserstoff erhalten werden,
G) Abtrennung von Wasser aus dem mindestens einen Kondensatstrom e1 und gegebenenfalls dem Kondensatstrom f1 durch Phasentrennung, wobei mindestens ein C₄-Kohlenwasserstoffstroms g1 enthaltend n-Butan und Butadien und mindestens ein Abwasserstrom g2 erhalten werden,
H) Auftrennung des C₄-Kohlenwasserstoffstroms g1 in einen n-Butan enthaltenden Rückführstrom h1 und einen im Wesentlichen aus Butadien bestehenden Produktstrom h2 und Rückführung des Stroms h1 in die erste Dehydrierzone.

2. Verfahren nach Anspruch 1, **dadurch gekennzeichnet, dass** die nichtkatalytische Dehydrierung von n-Butan autotherm unter Einspeisung eines sauerstoffhaltigen Gases mit einem Sauerstoffgehalt von mindestens 90 Vol.-% durchgeführt wird.

3. Verfahren nach Anspruch 1 oder 2, **dadurch gekennzeichnet, dass** der n-Butan enthaltende Einsatzstrom a aus liquefied petroleum gas (LPG) gewonnen wird.

4. Verfahren nach einem der Ansprüche 1 bis 3, **dadurch gekennzeichnet, dass** in Schritt C) und gegebenenfalls B) als sauerstoffhaltiges Gas technisch reiner Sauerstoff eingespeist wird.

5. Verfahren nach einem der Ansprüche 1 bis 5, **dadurch gekennzeichnet, dass** in Schritt H) eine Extraktivdestillation durchgeführt wird.

6. Verfahren nach einem der Ansprüche 1 bis 5, **dadurch gekennzeichnet, dass** nach Durchführung von Schritt C) in dem Gasstrom c enthaltener Restsauerstoff entfernt wird.

## Claims

1. A process for preparing butadiene from n-butane, which comprises the steps
A) provision of a feed gas stream a comprising n-butane;
B) feeding of the feed gas stream a comprising n-butane into at least one first dehydrogenation zone and nonoxidative, catalytic dehydrogenation of n-butane to give a gas stream b comprising n-butane, 1-butene, 2-butene, butadiene, hydrogen, possibly carbon dioxide and possibly water vapor;
C) feeding of the gas stream b and an oxygen-comprising gas having an oxygen content of at least 75% by volume into at least one second dehydrogenation zone and oxidative dehydrogenation of 1-butene and 2-butene to give a gas stream c comprising n-butane, butadiene, hydrogen, carbon dioxide and water vapor,
D) compression in at least one first compression stage and cooling of the gas stream c to give at least one condensate stream d1 comprising water and a gas stream d2 comprising n-butane, butadiene, hydrogen, carbon dioxide and water vapor,
E) compression in at least one further compression stage and cooling of the gas stream d2 to give at least one condensate stream e1 comprising n-butane, butadiene and water and a gas stream e2 comprising n-butane, butadiene, hydrogen and carbon dioxide,
F) cooling of the gas stream e2 to give a condensate stream f1 comprising n-butane and butadiene and an offgas stream f2 comprising carbon dioxide and hydrogen,
G) separation of water from the at least one condensate stream e1 and, if appropriate, the condensate stream f1 by phase separation to give at least one C₄-hydrocarbon stream g1 comprising n-butane and butadiene and at least one wastewater stream g2,
H) fractionation of the C₄-hydrocarbon stream g1 to give a recycle stream h1 comprising n-butane and a product stream h2 consisting essentially of butadiene and recirculation of the stream h1 to the first dehydrogenation zone.

2. The process according to claim 1, wherein the noncatalytic dehydrogenation of n-butane is carried out autothermally with introduction of an oxygen-comprising gas having an oxygen content of at least 90% by volume.

3. The process according to claim 1 or 2, wherein the feed stream a comprising n-butane is obtained from liquefied petroleum gas (LPG).

4. The process according to any of claims 1 to 3, wherein technically pure oxygen is fed in as oxygen-comprising gas in step C) and, if appropriate, B).

5. The process according to any of claims 1 to 5, wherein an extractive distillation is carried out in step H).

6. The process according to any of claims 1 to 5, wherein residual oxygen comprised in the gas stream c is removed after step C) has been carried out.

## Revendications

1. Procédé pour la fabrication de butadiène à partir de n-butane, comprenant les étapes suivantes :
A) on prépare un courant de gaz d'utilisation a contenant du n-butane ;
B) on injecte le courant de gaz d'utilisation a, contenant du n-butane, dans au moins une première zone de déshydrogénation, on effectue une déshydrogénation catalytique non oxydante, ce qui forme un courant de gaz b contenant du n-butane, du 1-butène, du 2-butène, du butadiène, de l'hydrogène, éventuellement, du dioxyde de carbone et, éventuellement, de la vapeur d'eau ;
C) on injecte le courant de gaz b et un gaz contenant de l'oxygène, dont la teneur en oxygène est d'au moins 75 % en volume, dans au moins une seconde zone de déshydrogénation, et on effectue une déshydrogénation oxydante du 1-butène et du 2-butène pour obtenir un courant de gaz c contenant du n-butane, du butadiène, de l'hydrogène, du dioxyde de carbone et de la vapeur d'eau,
D) on comprime le courant de gaz c dans au moins une première étape de compression et on le refroidit de manière à obtenir au moins un courant de condensat d1, contenant de l'eau, et un courant de gaz d2, contenant du n-butane, du butadiène, de l'hydrogène, du dioxyde de carbone et de la vapeur d'eau,
E) on comprime le courant de gaz d2 dans au moins une autre étape de compression, et on le refroidit de manière à obtenir au moins un courant de condensat e1 contenant du n-butane, du butadiène et de l'eau, et un courant de gaz e2 contenant du n-butane, du butadiène, de l'hydrogène et du dioxyde de carbone,
F) on refroidit le courant de gaz e2, ce qui forme un courant de condensat f1 contenant du n-butane et du butadiène, et un courant de gaz d'évacuation f2 contenant du dioxyde de carbone et de l'hydrogène,
G) on sépare l'eau du au moins un courant de condensat e1 et, éventuellement, du courant de condensat f1, par le biais d'une séparation de phases, de sorte que l'on obtienne au moins un courant d'hydrocarbure en C₄ g1, contenant du n-butane et du butadiène, et au moins un courant d'eau d'évacuation g2,
H) on sépare le courant d'hydrocarbure en C₄ g1 de manière à obtenir un courant de recyclage h1, contenant du n-butane, et un courant de produits h2, contenant essentiellement du butadiène, et on renvoie le courant h1 dans la première zone de déshydrogénation.

2. Procédé selon la revendication 1, **caractérisé en ce que** la déshydrogénation non catalytique du n-butane est effectuée en mode autothermique par injection d'un gaz contenant de l'oxygène, dont la teneur en oxygène est d'au moins 90 % en volume.

3. Procédé selon les revendications 1 ou 2, **caractérisé en ce que** le courant d'utilisation a, contenant du n-butane, provient de gaz de pétrole liquéfié (GPL).

4. Procédé selon l'une quelconque des revendications 1 à 3, **caractérisé en ce que** l'on injecte à l'étape C) et, éventuellement, à l'étape B), de l'oxygène de pureté technique comme gaz contenant de l'oxygène.

5. Procédé selon l'une quelconque des revendications 1 à 5, **caractérisé en ce que** l'on effectue une distillation extractive à l'étape H).

6. Procédé selon l'une quelconque des revendications 1 à 5, **caractérisé en ce que**, après avoir effectuée l'étape C), on élimine l'oxygène résiduel contenu dans le courant de gaz c.
